# EUROPEAN PATENT APPLICATION

(11) **EP 4 434 590 A1**
(43) Date of publication of application: **25.09.2024**
(21) Application number: 23382261.8
(22) Date of filing: 21.03.2023
(51) Int. Cl.: A61Q 19/00, A61Q 19/08, A61Q 17/00, A61K 8/06, A61K 8/9728, A61K 8/34, A61K 8/37, A61K 8/365

(54) **COSMETIC ACTIVE INGREDIENT COMPRISING AN EXTRACT OF THE ENDOPHYTIC YEAST KWONIELLA MANGROVIENSIS AND USES THEREOF**

(71) Applicant: Provital, S.A., 08210 Barberà del Vallès (ES)
(72) Inventor: MANZANO ALÍAS, David, 08025 BARCELONA (ES); ASO PÉREZ, Miguel, 08191 RUBÍ (ES); ESCUDERO FERNÁNDEZ, Elizabeth, 43840 SALOU (ES)
(74) Representative: Sugrañes, S.L.P.

(57) **Abstract**

The present invention refers to a cosmetic active ingredient comprising an extract of the endophytic yeast *Kwoniella mangroviensis* and uses thereof. It refers also to a cosmetic composition comprising said cosmetic active ingredient, and to a cosmetic method for treating the skin. The cosmetic active ingredient improves the microbiome balance of the skin, protects against the negative effects of pollution and produces a positive impact on the extracellular matrix of fibroblasts and on upregulating anti-aging genes and downregulating pro-aging genes.

## Description

### FIELD OF THE INVENTION

The present invention relates to the field of compositions for the topical application on the skin in order to improve the state of the skin, preferably human skin.

### BACKGROUND ART

The skin is the largest organ of the body. It is a complex organ that performs numerous vital functions, such as protection, thermoregulation and sensation. One of the main functions is to act as a protective barrier between the outside and the inside of the body, by protecting from external factors such as bacteria, chemicals and temperature. The skin is a good indicator of the general health.

The skin is a complex barrier organ which is home to a number of microorganisms (bacteria, fungi, viruses) on its surface, as disclosed in Byrd et al., The human skin microbiome, Nat. Rev. Microbiol., 2018, 16(3), 143-155. This microflora colonizing the stratum corneum constitutes the skin microbiome. Its composition and abundance are influenced by genetics and lifestyle. Microorganisms and their host live in perfect harmony. Skin microbiome influences immunity, nutrition and defense against external or pathogenic attacks; this symbiosis between the skin and its microbiome is beneficial and essential to its health and beauty.

However, the balance in the skin flora is constantly threatened by various factors, intrinsic and extrinsic, which can alter the composition of the microbiome and the barrier function of the skin. For example, skin aging causes an imbalance in the skin flora (dysbiosis), which leads to the appearance of disorders in the skin and to the deterioration of the state (health and beauty) of the skin.

Additionally, the evidence on the role of air pollution on skin aging has increased in recent years and the association between traffic-related air pollution and skin aging has been well-established, as disclosed in Schikowski et al., Air pollution and skin aging, Curr. Environ. Health Rep., 2020, 7(1), 58-64.

Different approaches have been described in the art to minimize the effects of aging on the skin.

For example, in the review Csekes et al., Skin Aging, Cellular Senescence and Natural Polyphenols, Int. J. Mol. Sci., 2021, 22(23), 12641, it is disclosed that skin aging is associated with an onset of age-related skin disorders and diseases, including dermatoses, infections and malignancies, besides triggering prominent visible manifestations, such as wrinkling, sagging, dryness or age spots, and that natural anti-senescence polyphenols have a great potential to be used in the prevention and treatment of the visible signs of premature and chronological aging and age-related disorders of the skin.

In the review Fania et al., Role of Nicotinamide in Genomic Stability and Skin Cancer Chemoprevention, Int. J. Mol. Sci., 2019, 20(23, 5946, it is disclosed that nicotinamide, an amide form of vitamin B3 is involved in the maintenance of genomic stability and may have beneficial effects against skin aging changes and tumour development, and that clinical studies showed that topical use of nicotinamide reduces cutaneous aging.

In the review Chen et al., Oxidative stress in the skin: Impact and related protection, Int. J. Cosmet. Sci., 2021, 43(5), 495-509, it is suggested that cutaneous microbiota play an indispensable role in skin quality influenced by pollutants and UV exposure. It is further disclosed that skin aging is a complex process comprising both intrinsic and extrinsic aging, wherein intrinsic aging is a 'natural' process, partly due to ROS (reactive oxygen species) accumulation, resulting from oxidative cellular metabolism, which is primarily determined by genetic factors, and extrinsic aging, on the other hand, is largely due to various environmental factors, such as sun and UV exposure, pollution smoking, and stress. To improve the capacity of antioxidant defenses it is proposed the use of antioxidants such as vitamin C, resveratrol and ferulic acid.

In the review Boxberger et al., Challenges in exploring and manipulating the human skin microbiome, Microbiome, 2021, 9(1): 125. doi:10.1186, it is disclosed that skin microbiota composition depends on intrinsic factors (skin site -biogeography factor, intra- and interpersonal variability, ethnicity, gender, aging) and on extrinsic factors (lifestyle, hygiene, geographical location, climate, seasonality). It is further disclosed that formulations containing a *Hylocereus undatus* fruit extract can reduce the perception of skin imperfections, and may positively influence the skin microbiota balance; and that bacterial extracts were also tested, such as an extract of *Shingomonas hydrophobicum* and lactobionic acid from *Pseudomonas taetrolens*, for antiaging activity.

Thus, in view that aging, pollution and dysbiosis are interconnected, there is still a need to provide further cosmetic active ingredients to improve the state of the skin, preferably, human skin.

### SUBJECT-MATTER OF THE INVENTION

The present invention relates to a cosmetic active ingredient comprising an extract of *Kwoniella mangroviensis.*

Another aspect of the invention relates to the cosmetic use of said cosmetic active ingredient.

Another aspect of the invention relates to a cosmetic composition comprising said cosmetic active ingredient.

Another aspect of the invention relates to a cosmetic method for treating the skin.

### FIGURES

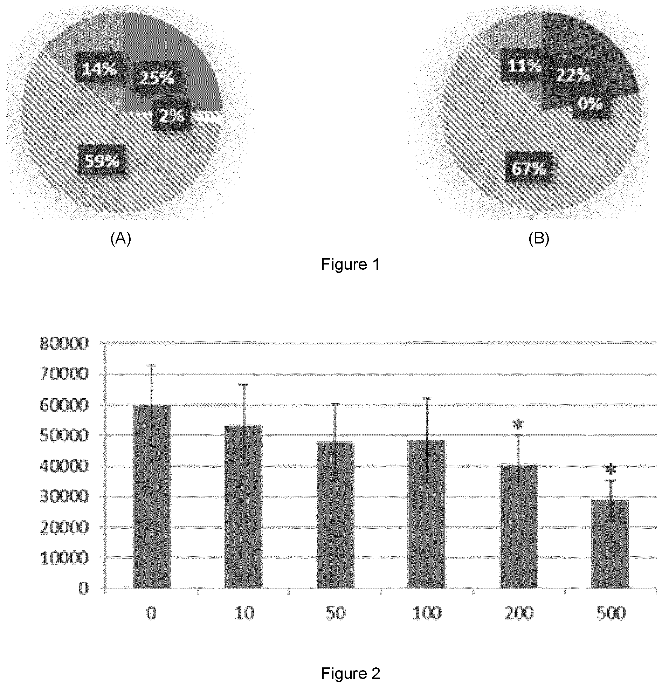

Figure 1 shows the quantification of the contribution of each of the microorganism species to the total microbial population, expressed as % of the total in cfu/cm², before the treatment with the cosmetic active ingredient of the invention (Figure 1 (A)), and after said treatment (Figure 1 (B)), as disclosed in Example 2. It can be observed that the relative abundance of *S*. *epidermidis* increased from 59% to 67%, while the relative abundance of *C*. *striatum* decreased from 25% to 22%, and *C*. *acnes* decreased from 2% to 0%. The relative abundance of *S*. *aureus* decreased from 14% to 11%.
Figure 2 shows the viability of human keratinocytes in the presence of increasing doses of Urban Dust, expressed in µg/mL, as disclosed in Example 3. In ordinates the amount of ATP is expressed in relative light units (RLU). It can be observed a statistically significant decrease in viability at 200 µg/mL, although the trend is evident even at lower doses.

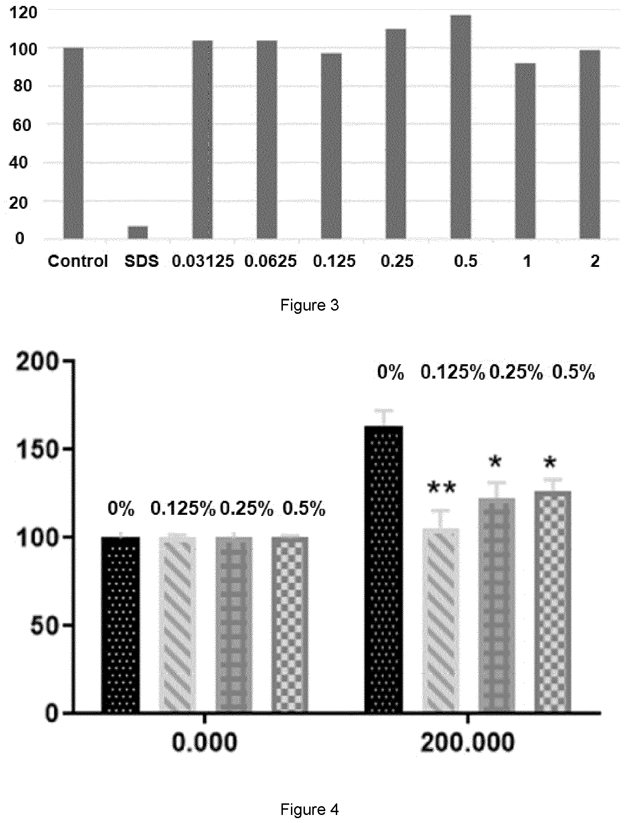

Figure 3 shows the viability of human keratinocytes in the presence of increasing doses of the cosmetic active ingredient of the invention, expressed in %, as disclosed in Example 3. In ordinates the viability is expressed as percentage versus the control. It can be observed that said cosmetic active ingredient does not induce any decrease in the viability of these cells.
Figure 4 shows the effect of different percentages (0%, 0.125%, 0.25% and 0.5%) of the cosmetic active ingredient of the invention on the formation of ROS, represented in ordinates as % vs Control, at 0 µg/mL and 200 µg/mL Urban Dust, as disclosed in Example 3. It can be observed a significant prevention in the three doses tested.

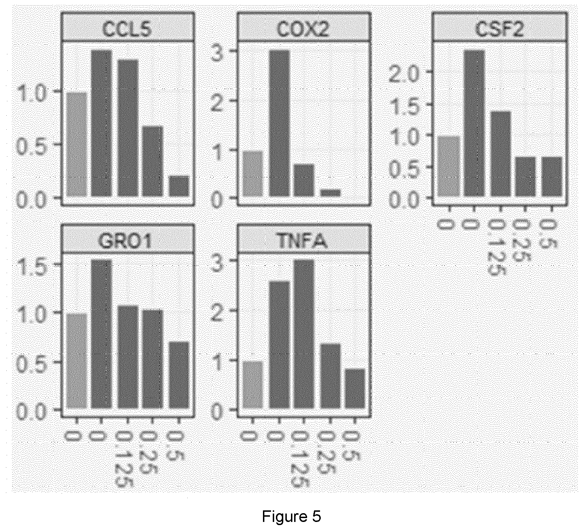

Figure 5 shows the effect of different percentages (0%, 0.125%, 0.25% and 0.5%) of the cosmetic active ingredient of the invention on the change in the generation of inflammatory cytokines, determined by the change in the following inflammatory genes: CCL5, COX2, CSF2, GRO1 and TNFA. In ordinates, the fold change is represented, and in abscises, different percentages of said active ingredient are represented as black columns, whereas the grey column represents the control, not treated with Urban Dust, as disclosed in Example 3. It can be observed that dose Increasing levels of active prevent markedly the expression of these 5 inflammatory cytokine genes.

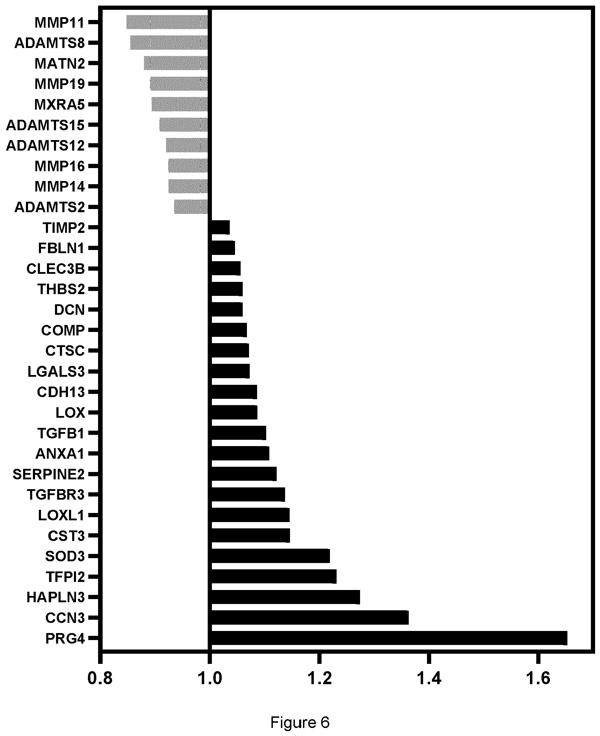

Figure 6 shows the expression levels of genes related to the extracellular matrix, as disclosed in Example 4. Expression levels are indicated in ordinates as fold change (FC) in active-treated Hayflick HDF cells (bars) relative to expression levels in the same cells untreated with active (control) (vertical line, FC=1). The genes whose expression increases during the natural aging of the extracellular matrix are shown in black, and the genes that decrease as a consequence of this process are shown in grey. It can be observed that there is a correlation between the direction of the observed change and an antiaging effect of the asset. Thus, genes whose expression increases during the natural aging of the extracellular matrix are repressed by the effect of the active ingredient (black bars in the figure) and, conversely, genes whose expression decreases as a consequence of aging are induced as a consequence of treatment with the asset (grey bars).

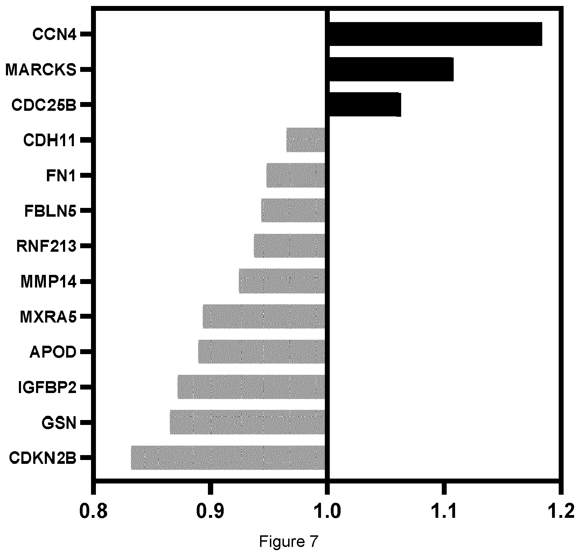

Figure 7 shows the expression levels of genes related to the senescence process, as disclosed in Example 4. Expression levels are indicated as fold change (FC) in active-treated Hayflick HDF cells (bars) relative to expression levels in the same cells untreated with active (control) (vertical line, FC=1). The genes whose expression increases as a consequence of the senescence process are shown in black, and the genes whose expression decreases as a consequence of said process are shown in grey.

### DESCRIPTION OF THE INVENTION

The object of the present invention is a cosmetic active ingredient comprising an extract of *Kwoniella mangroviensis.*

The inventors of the present invention have developed a cosmetic active ingredient comprising an extract of *Kwoniella mangroviensis,* which, surprisingly, shows a positive effect on the skin microbiome increasing the relative abundance of *Staphylococcus epidermidis* to the detriment of the relative abundance of *Staphylococcus aureus, Corynebacterium striatum* and *Cutibacterium acnes*, strains associated to opportunistic infections or to the development of processes such as acne, respectively. Said extract also shows a protective effect on skin against extern injuries caused by pollution and an anti-aging effect.

In the present description, as well as in the claims, the singular forms "a", "an" and "the" include the plural reference unless the context clearly indicates otherwise. The term "about" refers to a deviation of plus/minus 10%, preferably plus/minus 5%. The percentages are expressed in % by weight (wt%), unless stated the contrary. The ranges defined by the terms "between ... and ..." or by the terms "from ...to..." are meant to include also said stated endpoints thereof, and they also include any narrower sub-range.

### Extract of Kwoniella mangroviensis

In the present description, an extract means a preparation of a substance obtained from plants, animals, bacteria, fungi or yeast by means of a technical process, and used as an active ingredient.

In the present description, a lysate means any extract derived from cells obtained by a method comprising at least an enzymatic hydrolysis, a chemical hydrolysis. an osmotic disruption or a mechanical disruption step of the plasma membrane of a population of said cells. In a particular embodiment the cells are yeast cells.

*Kwoniella mangroviensis,* also known as *Kwoniella europaea,* is a yeast isolated from cork oak trees.

In a preferred embodiment of the invention, it is used the *Kwoniella mangroviensis* strain deposited with the number CECT 11955 (*Colección Española de Cultivos Tipo*, Dra. Carmela Belloch, CSIC, 2001).

References to this strain and other strains of *Kwoniella mangroviensis* are found in Villa-Carvajal et al., Polyphasic identification of yeasts isolated from bark of cork oak during the manufacturing process of cork stoppers, FEMS Yeast Res., 2004, 4, 745-750; Statzell-Tallman et al., Kwoniella mangroviensis gen. nov., sp. nov. (Tremellales, Basidiomycota), a teleomorphic yeast from mangrove habitats in the Florida Everglades and Bahamas, FEMS Yeast Res., 2008, 8, 103-113, and Guerreiro et al., Molecular and Genetic Evidence for a Tetrapolar Mating System in the Basidiomycetous Yeast Kwoniella mangroviensis and Two Novel Sibling Species, Eukar. Cell., 2013, 12(5), 746-760.

Said *Kwoniella* yeast is considered an endophyte. An endophyte is an endosymbiont that lives within a plant for at least part of its life cycle without causing apparent disease.

In the prior art there are not disclosures on the use of the yeast *Kwoniella mangroviensis* or any extract thereof in cosmetics. Surprisingly, the cosmetic active ingredient comprising an extract thereof is capable of improving the microbiome balance of the skin, protecting against the negative effects of pollution and producing a positive impact on the extracellular matrix of fibroblasts and on upregulating anti-aging genes and downregulating pro-aging genes.

The extract of *Kwoniella mangroviensis* is obtained by a process comprising culturing cells of said yeast in a culture medium suitable for the development of said cells in a conventional manner for a person skilled in the art to obtain a biomass of *Kwoniella mangroviensis.*

Culture media are selected among broth suitable for maintaining and propagating yeast strains such as disclosed in, for example, Hahn-Hägerdahl et al., Role of cultivation media in the development of yeast strains for large scale industrial use, Microb. Cell Fact., 2005, 4(31), doi:10.1186/1475-2859-4-31, or in Green et al., Media and Culture of Yeast, Curr. Protocols Cell Biol., 2001, doi:10.1002/0471143030.cb0106s04: YP (yeast extract and peptone), DM (defined mineral medium), YPD (yeast extract, peptone, and G-glucose), YNB (yeast extract ammonium base), YPG (yeast extract, peptone and glycerol), YPDG (yeast extract, peptone, dextrose, and glycerol) which can be supplemented with buffers, vitamins, trace elements, amino acids, or mixtures thereof.

In a preferred embodiment, the culture medium comprises yeast extract, which is a mixture of amino acids, peptides, water soluble vitamins and carbohydrates.

In the scope of the invention, the extract of *Kwoniella mangroviensis* comprises compounds produced by the yeast, which are present in the supernatant, and compounds obtained from the yeast itself, which are present in the biomass of *Kwoniella mangroviensis.* The latter compounds are completely dependent on the specific yeast strain, whereas the former compounds may possibly be produced by other types of yeast.

In an embodiment, the extract of *Kwoniella mangroviensis* comprises a supernatant and a lysate of the yeast cells obtained by culturing said cells in a culture medium.

In a preferred embodiment, the supernatant comprises the culture medium.

In a more preferred embodiment, the extract of *Kwoniella mangroviensis* comprises a supernatant, a lysate of the yeast cells obtained by culturing said cells in a culture medium, and the culture medium.

In an embodiment, the lysate of the yeast cells is obtained by mechanical disruption, enzymatic hydrolysis or chemical hydrolysis.

In a preferred embodiment, the lysate of the yeast cells of *Kwoniella mangroviensis* is obtained by a method comprising a chemical hydrolysis, more preferably an alkaline chemical hydrolysis.

In an embodiment, the extract comprises between 10 and 4000 ppm, preferably between 20 and 3000 ppm, more preferably between 30 and 2000 ppm, more preferably between 50 and 1000 ppm, more preferably between 70 and 700 ppm, and yet more preferably 100 and 400 ppm of xylitol; and between 50 and 10000 ppm, preferably between 100 and 6000 ppm, more preferably between 200 and 4000 ppm, more preferably between 300 and 3000 ppm, more preferably between 400 and 2000 ppm, and yet more preferably between 500 and 1000 ppm of β-glucan.

Usually, the extract comprises about 95 wt% of water, and the dry residue is about 2 wt% - 3 wt%.

In an embodiment, the extract further comprises an additive. Generally, the additive is selected from pentylene glycol, levulinic acid, glyceryl caprylate, sodium benzoate, potassium benzoate, sodium propionate, potassium sorbate, gluconolactone (δ-lactone of gluconic acid), lactic acid, tartaric acid, and mixtures thereof; preferably the additive is selected from pentylene glycol, levulinic acid, glyceryl caprylate, and mixtures thereof. In a preferred embodiment, the additive is a mixture of pentylene glycol, levulinic acid, and glyceryl caprylate. In a more preferred embodiment, the extract comprises the mixture of 2.5 wt%- 3.5 wt% pentylene glycol, 0.3 wt% - 0.4 wt% levulinic acid and 0.1 wt% - 0.16 wt% glyceryl caprylate as additives.

### Cosmetic active ingredient

In the present description, a cosmetic active ingredient means a compound or a set of compounds having a cosmetic effect on the skin, in particular on the skin cells.

The cosmetic active ingredient of the present invention comprises generally between 30 wt% and 90 wt% of the extract of *Kwoniella mangroviensis,* preferably between 35 wt% and 85 wt%, more preferably between 40 wt% and 80 wt%, more preferably between 45 wt% and 75 wt%, more preferably between 50 wt% and 75 wt%, and yet more preferably between 55 wt% and 75 wt%, based on the total weight of the cosmetic active ingredient.

In an embodiment, the cosmetic active ingredient comprises the extract of *Kwoniella mangroviensis* and further a cosmetically acceptable component selected from co-solvents, thickeners, additives, and mixtures thereof.

In an embodiment, the cosmetic active ingredient comprises a co-solvent. Generally, the co-solvent is selected from 1,3-propanediol, 1,2-propanediol (propylene glycol), polyethylene glycol, glycerin, and mixtures thereof, preferably the co-solvent is 1,3-propanediol. The content of the co-solvent is generally comprised between 10 wt% and 70 wt%, preferably between 15 wt% and 65 wt%, more preferably between 20 wt% and 60 wt%, more preferably between 25 wt% and 55 wt%, more preferably between 25 wt% and 50 wt%, and yet more preferably between 25 wt% and 45 wt%, based on the total weight of the cosmetic active ingredient.

In an embodiment, the cosmetic active ingredient comprises a thickener. Generally, the thickener is selected from xanthan gum, cross-linked acrylic acid (carbomer), ethyl cellulose, hydroxyethyl cellulose, hypromellose, carboxymethyl cellulose sodium, carrageenan, chitosan, guar gum, tara guam, scleroglucan, bentonite, hectorite, pectin, sodium alginate, and mixtures thereof; preferably the thickener is xanthan gum. The content of the thickener is generally comprised between 0.1 wt% and 1 wt%, preferably between 0.2 wt% and 0.9 wt%, more preferably between 0.25 wt% and 0.80 wt%, and yet more preferably between 0.3 wt% and 0.6 wt%, based on the total weight of the cosmetic active ingredient.

In an embodiment, the cosmetic active ingredient comprises an additive. Generally, the additive is selected from pentylene glycol, levulinic acid, glyceryl caprylate, sodium benzoate, potassium benzoate, sodium propionate, potassium sorbate, gluconolactone (δ-lactone of gluconic acid), lactic acid, tartaric acid, and mixtures thereof; preferably the additive is selected from pentylene glycol, levulinic acid, glyceryl caprylate, and mixtures thereof. In a preferred embodiment, the additive is a mixture of pentylene glycol, levulinic acid, and glyceryl caprylate. The content of the additive is generally comprised between 1 wt% and 7 wt%, preferably between 1.5 wt% and 6.5 wt%, more preferably between 2 wt% and 5.5 wt%, and yet more preferably between 2.5 wt% and 5 wt%, based on the total weight of the cosmetic active ingredient. In a preferred embodiment, the cosmetic active ingredient comprises between 0.5 wt% and 6 wt%, preferably between 1 wt% and 5.5 wt%, more preferably between 1.5 wt% and 5 wt%, more preferably 2 wt% and 4.5 wt%, and yet more preferably between 2.5 wt% and 3.5 wt%, based on the total weight of the cosmetic active ingredient, of pentylene glycol; between 0.1 wt% and 0.8 wt%, preferably between 0.2 wt% and 0.7 wt%, more preferably between 0.2 wt% and 0.6 wt%, more preferably 0.3 wt% and 0.5 wt%, and yet more preferably between 0.3 wt% and 0.4 wt%, based on the total weight of the cosmetic active ingredient, of levulinic acid; and between 0.01 wt% and 0.5 wt%, preferably between 0.02 wt% and 0.4 wt%, more preferably between 0.03 wt% and 0.3 wt%, more preferably 0.05 wt% and 0.2 wt%, and yet more preferably between 0.1 wt% and 0.16 wt%, based on the total weight of the cosmetic active ingredient, of glyceryl caprylate.

In a preferred embodiment, the cosmetic active ingredient comprises the extract of *Kwoniella mangroviensis,* a co-solvent, preferably, 1,3-propanediol, a thickener, preferably, xanthan gum, and as additive, preferably a combination of pentylene glycol, levulinic acid, and glyceryl caprylate.

The cosmetic active ingredient comprises generally the following components, expressed in wt% on dry matter: between 75 wt% and 85 wt% of carbohydrates, between 7 wt% and 15 wt% of proteins, and between 7 wt% and 15 wt% of ashes, wherein the sum of the components is 100%.

The determination of the carbohydrate content may be carried out, for example, according to the Dubois method as disclosed in Dubois et al., Colorimetric Method for Determination of Sugars and Related Substances, Anal. Chem., 1956, 28(3), 350-356, or according to the Luff-Schoorl method as disclosed in ISA 28-1e (Revision LT 22.01.2002) of the International Starch Institute (Denmark).

The determination of the protein content may be carried out according to the method as disclosed in Lowry et al., Protein measurement with the Folin phenol reagent, J. Biol. Chem., 1951, 193(1), 265-75, or by the determination of the total nitrogen content according to the well-known Kjeldahl method.

The determination of the ash content may be carried out by weighing the residues from the incineration of the samples of the cosmetic active ingredient of the invention at 700°C in an electric muffle furnace for about 1 h 30 min.

### Cosmetic uses

The cosmetic active ingredient is particularly effective for a cosmetic, non-therapeutic treatment of the skin.

An aspect of the invention is a cosmetic use of the cosmetic active ingredient for dermal application for improving the state of the skin.

In the scope of the invention, the term "improving the state of the skin" means i) the induction of a positive balance in the microbiome of the skin, ii) the protection of the skin from extern injuries caused by pollution, and/or iii) the reversion of the effects caused by ageing.

The induction of a positive balance in the microbiome of the skin is the result of increasing the relative abundance of beneficial commensals and reducing the relative abundance of pathogen commensals in the microbiome;

The protection of the skin from extern injuries caused by pollution is the result of reducing the content of reactive oxygen species and by preventing the rise of inflammatory cytokines, both generated by the treatment of keratinocytes with urban dust.

The reversion of the effects caused by senescence is the result of inducing the expression of genes related to the organization, structure and composition of the extracellular matrix, and by reversing the effects caused by senescence in gene expression levels, wherein the genes are related to the processes of general senescence, and to the specific senescence in the skin.

In an embodiment, the cosmetic use of the cosmetic active ingredient is selected from inducing a positive balance in the microbiome of the skin, protecting the skin from extern injuries caused by pollution, reversing the effects caused by senescence, and combinations thereof.

In an embodiment, the cosmetic use of the cosmetic active ingredient is for inducing a positive balance in the microbiome of the skin.

In an embodiment, the cosmetic use of the cosmetic active ingredient is for protecting the skin from extern injuries caused by pollution.

In an embodiment, the cosmetic use of the cosmetic active ingredient is for reversing the effects caused by senescence, and combinations thereof.

### A) Effect of the cosmetic active ingredient on the skin microbiome

In an embodiment, the cosmetic use of the cosmetic active ingredient is for improving the state of the skin by inducing a positive balance in the microbiome of the skin. In a preferred embodiment the positive balance in the microbiome of the skin is an increase in the relative abundance of *Staphylococcus epidermidis*, and a decrease in the relative abundance of *Staphylococcus aureus, Cutibacterium acnes* and *Corynebacterium striatum* present in the microbiome of the skin.

A 3D *in vitro* reconstituted skin model may be used to assess the effect of the *Kwoniella mangroviensis* extract of the present invention on the skin microbiome.

This model, based on primary human cells, reconstitutes the dermis and epidermis and allows co-cultures with the main species of bacteria that colonize human skin: *S*. *epidermidis*, *S. aureus*, *C. atriatum* and *C*. *acnes.* These four species make up 90% of the human skin microbiome, making it a valid model to study the effect of cosmetic active ingredients on the skin microbiome. Once the skin tissue has been differentiated, the consortium of commensal bacteria made up of *S*. *epidermidis, C. atriatum* and *C*. *acnes* is applied first. After incubation for 24 hours, the tissue is inoculated with a pathogenic strain of *S*. *aureus* and incubated for a further 24 hours. The active ingredient is then added (at a final concentration of 2%) and it is incubated for another 24 hours before taking samples for analysis.

Samples of bacterial strains are collected for quantification and skin explants may be also collected for histological and gene expression analysis.

To evaluate the effect of the *Kwoniella mangroviensis* extract on the microbiome, the strains are usually grown in specific media and the number of cfu/cm² of skin is quantified.

The results may be expressed as a relative abundance between the different species for each of the treatments.

The results, shown in Example 2, show that the relative abundance of S. epidermidis increases by 7.95%, while the relative abundance of C. striatum and C. acnes decreases by 2.69% and 1.93%, respectively, as shown in Figure 1.

It is known that an increase in these latter species is associated with alterations in the healthy state of the skin, either due to opportunistic infections (case of *C*. *striatum*), or due to the development of processes such as acne (case of *C*. *acnes*)*.* The relative abundance of *S*. *aureus* decreases by 3.34%.

Thus, the cosmetic active ingredient of the invention is capable of acting on the skin microbiome, and, in particular, reducing the abundance of opportunistic or pathologic microorganisms and promoting the abundance of beneficial commensals.

These results show that the active promotes the appearance of a positive balance in the microbiome, which is associated with skin health and beauty.

### B) Effect of the cosmetic active ingredient to protect the skin from extern injuries caused by pollution.

In an embodiment, the cosmetic use of the cosmetic active ingredient is for improving the state of the skin by protecting skin from extern injuries caused by pollution. In a preferred embodiment, the protection of the skin is by reducing the content of reactive oxygen species generated by the treatment of keratinocytes with urban dust. In another preferred embodiment, the protection of the skin is by preventing the rise of inflammatory cytokines generated by the treatment of keratinocytes with urban dust.

The effect of the *Kwoniella mangroviensis* extract to protect the skin from extern injuries caused by pollution may be assessed by studying the viability of keratinocytes in the presence of increasing concentrations of Urban Dust (SMR1649b, reference material, National Institute of Standards and Technology, NIST, USA), as disclosed in Fernández et al., "SIG-1273 protects skin against urban air pollution and when formulated in AgelQTM Night Cream anti-aging benefits clinically demonstrated," J. Cosmet. Dermatol., 2019, doi: 10.1111/jocd.12825.

Urban Dust induces toxicity in human keratinocytes in a dose-dependent manner, exhibiting a statistically significant decrease in viability from a dose of 200 µg/ml; although the trend is evident at lower doses, as shown in Figure 2.

The *Kwoniella mangroviensis* extract itself does not induce toxicity in human keratinocytes. Thus, as shown in Figure 3, the *Kwoniella mangroviensis* extract does not induce any decrease in the viability of these cells.

The measurements of ROS and inflammatory mediators are suitable to assess the effect of the extract to protect the skin from extern injuries caused by pollution. As shown in Example 3, Urban Dust at 200 µg/ml induces an increase, with respect to the unstimulated control, of 164% in the formation of ROS. However, when the extract is used together with Urban Dust, a statistically significant prevention is observed in all the tested doses, as shown in Figure 4.

The protection of the skin by the extract of the present invention may also be seen by the prevention of rise in inflammatory mediators, induced by Urban dust. Using the RT-PCR technique, it is possible to measure a panel of inflammatory cytokines. Urban Dust induced large increases in the 5 inflammatory genes CCL5, COX2, CSF2, GRO1 and TNFA. When co-stimulating with Urban Dust and the active ingredient, it is observed that increasing levels of the extract prevent markedly the expression of these 5 inflammatory cytokines, as disclosed in Example 3, and Figure 5.

### C) Anti-aging effect of the cosmetic active ingredient

In an embodiment, the cosmetic use of the cosmetic active ingredient is for improving the state of the skin by reversing the effects caused by senescence. In a preferred embodiment, the improvement is by inducing the expression of genes related to the organization, structure and composition of the extracellular matrix. In a preferred embodiment, the improvement is by reversing the effects caused by senescence in gene expression levels, wherein the genes are related to the processes of general senescence, and to the specific senescence in the skin.

To assess the anti-aging effect of the *Kwoniella mangroviensis* extract it may be used a massive sequencing experiment on human fibroblasts (HDF), both young HDF and models of aged fibroblasts, the Hayflick model (see Kim et al., Implications of time-series gene expression profiles of replicative senescence, Aging Cell, 2013, 12(4), 622-634, doi: 10.1111/acel.12087), and the hydrogen peroxide chemical induction model (see De Magalhães et al., Gene expression and regulation in H2O2-induced premature senescence of human foreskin fibroblasts expressing or not telomerase, Exp. Gerontol., 2004, 39(9) 1379-1389, doi: 10.1016/j.exger.2004.06.004). Said fibroblasts are treated with the extract (treated samples) or using the same cell models without treating with the extract (control samples). The extraction of RNA and massive sequencing, combined by the use of bioinformatic tools lead to the identification of genes that are altered by the treatment.

As disclosed in Example 4 and in Figure 6, the presence of the extract reverses the effects caused by senescence in gene expression levels, wherein the genes are related to the processes of general senescence, as disclosed in De Magalhães *et al.*, *op. cit.*, and Fridman et al., Critical pathways in cellular senescence and immortalization revealed by gene expression profiling, Oncogene, 2008, 27(46), 5975-5987, doi: 10.1038/ONC.2008.213, and specific senescence in the skin, as disclosed in Waldera Lupa et al., Characterization of Skin Aging-Associated Secreted Proteins (SAASP) Produced by Dermal Fibroblasts Isolated from Intrinsically Aged Human Skin, J. Invest. Dermatol., 2015, 135(8), 1954-1968, 2015, doi: 10.1038/jid.2015.120.

Further, as shown in Example 4 and Figure 7, the treatment with the extract induces the expression of genes related to the organization, structure and composition of the extracellular matrix.

### Cosmetic composition

An aspect of the invention is a cosmetic composition for topical application comprising the cosmetic active ingredient of the invention and at least one cosmetically acceptable component.

In an embodiment, the cosmetic composition contains at least one cosmetically acceptable component, which in the context of the invention, is selected from a cosmetically acceptable vehicle, an additional cosmetic active component, an additional cosmetic ingredient, and mixtures thereof.

As used herein, "cosmetically-acceptable" means that the product(s) or compound(s), which the term describes are suitable for use in contact with tissues (e.g., the skin) without undue toxicity, incompatibility, instability, irritation, allergic response, and the like. This term is not intended to limit the ingredient/product, which describes to use solely as a cosmetic.

A cosmetically acceptable vehicle (or carrier) is a vehicle in which the cosmetic ingredients are dissolved, emulsified, dispersed, or suspended. Said vehicle is selected from among water, a water-miscible non-aqueous vehicle, such as ethanol or isopropanol, and a water-immiscible non-aqueous vehicle, such as, for example, vegetable oil, fatty esters, medium chain triglycerides, or alkanes. Preferably the cosmetic composition includes water as a vehicle.

In an embodiment, the additional cosmetic active ingredient can be selected from: skin soothing and healing agents, skin anti-aging agents, skin moisturizing agents, anti-wrinkle agents, anti-atrophy agents, skin smoothing agents, antibacterial agents, antifungal agents, antimicrobial agents, anti-inflammatory agents, anti-pruriginous agents, external anaesthetic agents, keratolytic agents, free radicals scavengers, anti-seborrheic agents, antidandruff agents, the agents modulating the differentiation, proliferation or pigmentation of the skin and agents accelerating penetration, desquamating agents, depigmenting or propigmenting agents, tightening agents, agents stimulating the synthesis of dermal or epidermal macro molecules and/or preventing their degradation, agents stimulating the proliferation of fibroblasts and/or keratinocytes or stimulating the differentiation of keratinocytes, muscle relaxants; antipollution and/or anti- free radical agents, slimming agents, anticellulite agents, agents acting on the microcirculation, agents acting on the energy metabolism of the cells, cleaning agents, hair conditioning agents, hair styling agents, hair growth promoters, sunscreen and/or sunblock compounds, make-up agents, antiseptic agents, deodorant actives, essential oils, cosmetic astringents, anti-acne agents, anti-caking agents, anti-foaming agents, antioxidants, biological additives, enzymes, enzymatic inhibitors, enzyme-inducing agents, coenzymes, plant extracts, plant derivatives, plant tissue extracts, plant seed extracts, plant oils, botanical extracts, ceramides, peptides, cosmetic biocides, denaturants, astringents compounds, film formers, quaternary derivatives, agents increasing the substantivity, opacifying agents, skin bleaching and lightening agents, skin tanning agents, skin-conditioning agents, skin soothing and/or healing agents, skin treating agents, vitamins and derivatives thereof, peeling agents, moisturizing agents, lignans, UV absorbers, water-soluble sunscreen, antiperspirant, depilatory, fat soluble sunscreen, skin restructuring agent, anti-mycobacterial agents, anti-allergenic agents, anti-irritants, immune system boosting agents, immune system suppressing agents, insect repellents, staining agents, hypopigmenting agents, photostabilizing agents, and mixtures thereof.

In an embodiment, the additional cosmetic ingredient is preferably selected from: surfactants (emulsifiers), lipid compounds, emollients, factors of consistency, thickening agents, stabilizers, hydrotropes, preserving agents, essences, colorants, silicone compounds, fats, waxes, lecithins, phospholipids, UV sun protection factors, film-forming agents, self-tanners, firming agents, and mixtures thereof.

In a preferred embodiment, the cosmetic composition contains an additional cosmetic ingredient selected from the group consisting of surfactants (emulsifiers), lipid, emollient compounds, consistency factors, thickening agents, hydrotropes, preservatives, and mixtures thereof.

The physical form of the cosmetic composition for the use according to the invention is not important. Said composition may be made into a wide variety of product types that include but are not limited to solid and liquid compositions such as solutions, lotions, creams, gels, sticks, sprays, ointments, cleansing liquid washes and solid bars, shampoos, pastes, powders, foams, mousses, milks, oil-in water emulsions, water-in-oil emulsions, multiple emulsions (water/oil/water or oil/water/oil), microemulsions, nanoemulsions, dispersions, suspensions, or wipes.

The topical compositions useful in the present invention may be formulated as solutions. Solutions may preferably include an aqueous solvent (e.g., from about 75 wt% to about 95 wt% or from about 75 wt% to about 85 wt% of a cosmetically acceptable aqueous solvent). More preferably, such compositions may contain about 30 wt% solvent, although this may vary dependent upon the formulation. Such solvents may include ethanol, propylene glycol, polyethylene glycol, mixtures thereof and the like. In an embodiment, the topical composition useful in the present invention may be formulated as a solution containing an emollient. Such composition preferably contains from about 2 wt% to about 50 wt% of an emollient. As used herein, "emollients" refer to materials used for the prevention or relief of dryness, as well as for the protection of the skin.

A lotion may be made from a solution. Lotions typically contain from about 1 wt% to about 20 wt% of an emollient and from about 50 wt% to about 90 wt% of water.

Another type of product may be a cream. A cream typically comprises from about 5 wt% to about 50 wt% of an emollient and from about 45 wt% to about 85 wt% of water.

Another type of product may be an ointment. An ointment may be constituted of a simple base of vegetable oils or semi-solid hydrocarbons. An ointment may contain from about 2 wt% to about 100 wt% of an emollient, and from about 0.1 wt% to about 5 wt% of a thickening agent.

The topical compositions useful in the present invention may also be preferably formulated as emulsions. In an embodiment, the cosmetic composition contains water and a lipophilic phase and is presented in the form of emulsion or dispersion, such as, for example, the oil-in-water (O/W), water-in-oil (W/O) type, multiple emulsion (W/O/W or O/W/O), or PIT-type emulsion, or microemulsion. If the carrier is an emulsion, from about 1 wt% to about 10 wt% of the carrier should be made up one or more emulsifiers. Emulsifiers may be non-ionic, anionic, cationic, or amphoteric.

The topical composition useful in the present invention may be formulated as a gel. Suitable gelling agents for aqueous gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as vegetable oils, esters) include, but are not limited to, waxes, modified silicas, cellulose derivatives, polyamides, polyurethanes, and L-glutamic acid derivatives. Such gels typically comprise between about 0.1 wt% and 20 wt% of such gelling agents.

The topical composition useful in the present invention may also be formulated into a solid formulation (e.g., a wax-based stick, mascara, soap bar composition, powder, a wipe containing powder, or cosmetotextile).

Further examples of cosmetically acceptable ingredients and vehicles are cited in particular in the International Cosmetic Ingredient Dictionary and Handbook published by Cosmetic, Toiletry, and Fragrance Association (CTFA).

### Cosmetic method

An aspect of the invention is a cosmetic method for treating the skin, preferably human skin, comprising applying topically to the skin of a subject in need thereof of an effective amount of a cosmetic composition comprising the cosmetic active ingredient of the invention.

In an embodiment, the method for treating the skin, preferably human skin, is for improving the state of the skin selected from inducing a positive balance in the microbiome of the skin, protecting skin from extern injuries caused by pollution, reversing the effects caused by ageing, and combinations thereof.

Generally, the cosmetic composition comprising the cosmetic active ingredient of the invention is applied at least once a day for at least 15, 28 or 56 days. preferably twice between 15 and 28 days.

The present invention may be defined according to the following embodiments:
1.- A cosmetic active ingredient comprising an extract of *Kwoniella mangroviensis.*
2.- The cosmetic active ingredient according to embodiment 1, characterized in that the extract of *Kwoniella mangroviensis* comprises a supernatant and a lysate of the yeast cells obtained by culturing said cells in a culture medium.
3.- The cosmetic active ingredient according to embodiment 2, characterized in that the culture medium comprises yeast extract.
4.- The cosmetic active ingredient according to embodiment 2 or 3, characterized in that the supernatant comprises the culture medium.
5.- The cosmetic active ingredient according to embodiment 2, characterized in that the lysate of the yeast cells of *Kwoniella mangroviensis* is obtained by mechanical disruption, enzymatic hydrolysis or chemical hydrolysis.
6.- The cosmetic active ingredient according to embodiment 5, characterized in that the lysate of the yeast cells is obtained by a method comprising a chemical hydrolysis, preferably an alkaline chemical hydrolysis.
7.- The cosmetic active ingredient according to any one of embodiments 1 to 6, characterized in that the extract comprises between 10 and 4000 ppm, preferably between 20 and 3000 ppm, more preferably between 30 and 2000 ppm, more preferably between 50 and 1000 ppm, more preferably between 70 and 700 ppm, and yet more preferably 100 and 400 ppm of xylitol; and between 50 and 10000 ppm, preferably between 100 and 6000 ppm, more preferably between 200 and 4000 ppm, more preferably between 300 and 3000 ppm, more preferably between 400 and 2000 ppm, and yet more preferably between 500 and 1000 ppm of β-glucan.
8.- The cosmetic active ingredient according to any one of embodiments 1 to 7, characterized in that the extract comprises the extract comprises about 95 wt% of water.
9.- The cosmetic active ingredient according to any one of embodiments 1 to 8, characterized in that the extract further comprises an additive.
10.- The cosmetic active ingredient according to embodiment 9, characterized in that the additive is selected from pentylene glycol, levulinic acid, glyceryl caprylate, sodium benzoate, potassium benzoate, sodium propionate, potassium sorbate, gluconolactone (δ-lactone of gluconic acid), lactic acid, tartaric acid, and mixtures thereof.
11.- The cosmetic active ingredient according to embodiment 10, characterized in that the additive is selected from pentylene glycol, levulinic acid, glyceryl caprylate, and mixtures thereof.
12.- The cosmetic active ingredient according to embodiment 11, characterized in that the additive is a mixture of pentylene glycol, levulinic acid, and glyceryl caprylate.
13.- The cosmetic active ingredient according to embodiment 12, characterized in that the extract comprises the mixture of 2.5 - 3.5 wt% pentylene glycol, 0.3 - 0.4 wt% levulinic acid and 0.1 - 0.16 wt% glyceryl caprylate as additives.
14.- The cosmetic active ingredient according to any one of embodiments 1 to 13, characterized in that it comprises between 30 wt% and 90 wt% of the extract of *Kwoniella mangroviensis,* preferably between 35 wt% and 85 wt%, more preferably between 40 wt% and 80 wt%, more preferably between 45 wt% and 75 wt%, more preferably between 50 wt% and 75 wt%, and yet more preferably between 55 wt% and 75 wt%, based on the total weight of the cosmetic active ingredient.
15.- The cosmetic active ingredient according to any one of embodiments 1 to 14, characterized in that it further comprises a cosmetically acceptable component selected from co-solvents, thickeners, additives, and mixtures thereof.
16.- The cosmetic active ingredient according to embodiment 15, characterized in that it comprises a co-solvent.
17.- The cosmetic active ingredient according to embodiment 16, characterized in that the co-solvent is selected from 1,3-propanediol, 1,2-propanediol (propylene glycol), polyethylene glycol, glycerin, and mixtures thereof.
18.- The cosmetic active ingredient according to embodiment 17, characterized in that the co-solvent is 1,3-propanediol.
19.- The cosmetic active ingredient according to any one of embodiments 16 to 18, characterized in that the content of the co-solvent is comprised between 10 wt% and 70 wt%, preferably between 15 wt% and 65 wt%, more preferably between 20 wt% and 60 wt%, more preferably between 25 wt% and 55 wt%, more preferably between 25 wt% and 50 wt%, and yet more preferably between 25 wt% and 45 wt%, based on the total weight of the cosmetic active ingredient.
20.- The cosmetic active ingredient according to any one of embodiments 15 to 19, characterized in that it comprises a thickener.
21.- The cosmetic active ingredient according to embodiment 20, characterized in that the thickener is selected from xanthan gum, cross-linked acrylic acid (carbomer), ethyl cellulose, hydroxyethyl cellulose, hypromellose, carboxymethyl cellulose sodium, carrageenan, chitosan, guar gum, tara guam, scleroglucan, bentonite, hectorite, pectin, sodium alginate, and mixtures thereof.
22.- The cosmetic active ingredient according to embodiment 21, characterized in that the thickener is xanthan gum.
23.- The cosmetic active ingredient according to any one of embodiments 20 to 22, characterized in that the content of the thickener is comprised between 0.1 wt% and 1 wt%, preferably between 0.2 wt% and 0.9 wt%, more preferably between 0.25 wt% and 0.80 wt%, and yet more preferably between 0.3 wt% and 0.6 wt%, based on the total weight of the cosmetic active ingredient.
24.- The cosmetic active ingredient according to any one of embodiments 15 to 23, characterized in that it comprises an additive.
25.- The cosmetic active ingredient according to embodiment 24, characterized in that the additive is selected from pentylene glycol, levulinic acid, glyceryl caprylate, sodium benzoate, potassium benzoate, sodium propionate, potassium sorbate, gluconolactone (δ-lactone of gluconic acid), lactic acid, tartaric acid, and mixtures thereof.
26.- The cosmetic active ingredient according to embodiment 25, characterized in that the additive is selected from pentylene glycol, levulinic acid, glyceryl caprylate, and mixtures thereof.
27.- The cosmetic active ingredient according to embodiment 26, characterized in that the additive is a mixture of pentylene glycol, levulinic acid, and glyceryl caprylate.
28.- The cosmetic active ingredient according to any one of embodiments 15 to 27, characterized in that the content of the additive is comprised between 1 wt% and 7 wt%, preferably between 1.5 wt% and 6.5 wt%, more preferably between 2 wt% and 5.5 wt%, and yet more preferably between 2.5 wt% and 5 wt%, based on the total weight of the cosmetic active ingredient.
29.- The cosmetic active ingredient according to embodiment 27, characterized in that it comprises between 0.5 wt% and 6 wt%, preferably between 1 wt% and 5.5 wt%, more preferably between 1.5 wt% and 5 wt%, more preferably 2 wt% and 4.5 wt%, and yet more preferably between 2.5 wt% and 3.5 wt%, based on the total weight of the cosmetic active ingredient, of pentylene glycol; between 0.1 wt% and 0.8 wt%, preferably between 0.2 wt% and 0.7 wt%, more preferably between 0.2 wt% and 0.6 wt%, more preferably 0.3 wt% and 0.5 wt%, and yet more preferably between 0.3 wt% and 0.4 wt%, based on the total weight of the cosmetic active ingredient, of levulinic acid; and between 0.01 wt% and 0.5 wt%, preferably between 0.02 wt% and 0.4 wt%, more preferably between 0.03 wt% and 0.3 wt%, more preferably 0.05 wt% and 0.2 wt%, and yet more preferably between 0.1 wt% and 0.16 wt%, based on the total weight of the cosmetic active ingredient, of glyceryl caprylate.
30.- The cosmetic active ingredient according to embodiment 1, characterized in that it comprises the extract of *Kwoniella mangroviensis,* a co-solvent, preferably 1,3-propanediol, a thickener, preferably xanthan gum, and an additive, preferably a combination of pentylene glycol, levulinic acid, and glyceryl caprylate.
31.- The cosmetic active ingredient according to any one of embodiments 1 to 30, characterized in that it comprises the following components, expressed in wt% on dry matter: between 75 wt% and 85 wt% of carbohydrates, between 7 wt% and 15 wt% of proteins, and between 7 wt% and 15 wt% of ashes, wherein the sum of the components is 100%.
32.- A cosmetic use of the cosmetic active ingredient according to any one of embodiments 1 to 31 for dermal application for improving the state of the skin.
33.- The cosmetic use according to embodiment 32, characterized in that it is selected from for inducing a positive balance in the microbiome of the skin, protecting the skin from extern injuries caused by pollution, reversing the effects caused by ageing, and combinations thereof.
34.- A cosmetic composition for topical application comprising the cosmetic active ingredient according to any one of embodiments 1 to 31 and at least one cosmetically acceptable component.
35.- The cosmetic composition according to embodiment 34, characterized in that the cosmetically acceptable component is selected from a cosmetically acceptable vehicle, an additional cosmetic active component, an additional cosmetic ingredient, and mixtures thereof.
36.- A cosmetic method for treating the skin, preferably human skin, comprising applying topically to the skin of a subject in need thereof of an effective amount of a cosmetic composition comprising the cosmetic active ingredient according to any one of embodiments 1 to 31.
37.- The cosmetic method according to embodiment 36, characterized in that the method for treating the skin is for improving the state of the skin selected from inducing a positive balance in the microbiome of the skin, protecting skin from extern injuries caused by pollution, reversing the effects caused by ageing, and combinations thereof.
38.- The cosmetic method according to embodiment 36 or 37, characterized in that the cosmetic composition comprising the cosmetic active ingredient according to any one of embodiments 1 to 31 is applied at least once a day for at least 15, 28 or 56 days.
39.- The cosmetic method according to embodiment 38, characterized in that the cosmetic active ingredient according to any one of embodiments 1 to 31 is applied twice between 15 and 28 days.

### Examples

### Preparative example: Preparation of Kwoniella mangroviensis extract

A culture of *Kwoniella mangroviensis,* whose geographic origin is Spain, was grown up in a medium suitable for their development, comprising yeast extract, comprising a mixture of amino acids, peptides, water soluble vitamins and carbohydrates, in a conventional manner for a person skilled in the art, at about 25-30°C to an optical density of at least about 60-80. The culture was centrifugated at about 8800 g to separate the cell culture supernatant from the yeast cells.

The culture supernatant was filtered through a filter of 0.22 µm.

The cells were treated under alkaline conditions to obtain a product free of viable yeast cells. The product was neutralized with an organic acid, citric acid or lactic acid, to a pH value of between 4 and 6.

The culture supernatant and the neutralized product were mixed and adjusted to obtain an extract comprising between 100 and 400 ppm of xylitol and between 500 and 1000 ppm of β-glucans.

The water content of the extract is about 95 wt%.

The extract further comprised 2.5 - 3.5 wt% pentylene glycol, 0.3 - 0.4 wt% levulinic acid and 0.1 - 0.16 wt% glyceryl caprylate, as additives.

### Example 1: Cosmetic active ingredient comprising the Kwoniella mangroviensis extract

A composition comprising the *Kwoniella mangroviensis* extract obtained according to the process disclosed in the Preparative Example was prepared as follows.

Levulinic acid and glyceryl caprylate were heated at 40-45°C to get them melted.

The extract was mixed with propanediol and pentylene glycol, and melted levulinic acid and glyceryl caprylate were added thereto and stirred up to homogenization.

The mixture was homogenized using a high shear mixer, and 1,3-propanediol and xanthan gum were added to the homogenized mixture, and mixed.

The pH value vas adjusted to a value between 4.7 and 5.1 using citric acid.

The cosmetic active ingredient comprised the components as shown in Table I:

**TABLE I**

| Component | wt. % |
|---|---|
| Extract of *Kwoniella mangroviensis* (Example 1) | 55-75 |
| 1,3-propanediol | 25-45 |
| Pentylene glycol | 2.5-3.5 |
| Xanthan gum | 0.3-0.6 |
| Levulinic acid | 0.3-0.4 |
| Glyceryl caprylate | 0.1-0.16 |

The dry matter of the cosmetic active ingredient comprises 78.9 wt% of carbohydrates, 10.3 wt% of proteins, and 10.8 wt% of ashes.

The cosmetic active ingredient of the invention comprising the *Kwoniella mangroviensis* extract comprised further between 170 and 260 ppm xylitol, and between 375 and 550 ppm β-glucan.

### Example 2: Effect of the Kwoniella mangroviensis extract on the skin microbiome

A 3D *in vitro* reconstituted skin model, as disclosed in REFERENCE, was used to assess the effect of the *Kwoniella mangroviensis* extract, obtained according to the process disclosed in Preparative example, on the skin microbiome.

Primary adult human dermal fibroblasts were embedded in a fibrin matrix to produce dermal equivalents (DE). Primary neonatal human keratinocytes were then applied to the DE surface and cultured under fluid for 48 hours. The tissue was then cultured at the air-liquid interface (ALI) until a stratified epidermis formed. Incubation conditions for all cultures were 37°C in 5% (v/v) CO₂ at >95% relative humidity.

Explants were maintained in deep well plates for the duration of the study. *Cutibacterium acnes* NCTC 737 was grown for 5 days anaerobically at 37°C using furazolidone-reinforced clostridial agar (RCAF) medium. *Staphylococcus epidermidis* NCTC 11047 and *Corynebacterium striatum* NCTC 764 were cultured aerobically for 18-24 hours using Mueller-Hinton agar (MHA) and aerobic Corynebacteria agar (ACA), respectively. Inoculation buffer (GS25) was used along with bacteria from the cultures mentioned above to prepare the initial inoculum mix containing about 1.1 × 10⁶ cfu/mL of each bacterium (3x mix). 10 µL of the initial inoculum was pipetted and then spread with a glass rod on each skin unit to achieve a final concentration of ~104 cfu/cm² of each bacterium. The skin was incubated at about 37 °C in 5% CO2 (v / v) a> 95% de RH about24 hours.

On the day of colonization of the skin with 3x mix, *Staphylococcus aureus* strain NCTC 13435 was inoculated on MHA. Plates were incubated aerobically at about 37°C for 18-24 hours. Bacteria from a culture of *S*. *aureus* strain NCTC 13435 mentioned above were used to prepare a bacterial suspension in GS25 buffer. The skin was infected with 10² cfu/cm² of *S*. *aureus.* Infected skin was incubated at 37°C in 5% (v/v) CO² at >95% RH for about 24 hours. Colonized skin units were treated with 10 µL of active at a concentration of 2% (v/v) or left untreated as negative controls. Finally, colonized skin was incubated at 37°C in 5% (v/v) CO₂ at >95% relative humidity for about 24 hours.

Colony forming units (cfu) were analysed by recovery in appropriate culture media. Two solid media were used to count staphylococci: MHA and Baird-Parker Agar (BPA). The number of *S*. *epidermidis* was calculated by subtracting the number obtained from BPA from the number obtained from MHA. Selective media were used to count *C*. *acnes* or *C*. *striatum,* respectively. The rest of the insert tissue was then cut and the membrane was carefully removed from the insert.

The analysis showed that the relative abundance of *S*. *epidermidis* increased by about 8%, while the relative abundance of *C*. *striatum* and *C*. *acnes* decreased by about 3% and about 2%, respectively.

It has been described that an increase in these species has been associated with alterations in the healthy state of the skin, either due to opportunistic infections (case of *C*. *striatum*), or due to the development of processes such as acne (case of *C*. *acnes*)*.* The relative abundance of S. *aureus* decreases by about 3%.

These results showed that the extract promotes the appearance of a positive balance in the microbiome, frequently associated with skin health.

### Example 3: Effect of the Kwoniella mangroviensis extract to protect the skin from extern injuries caused by pollution

To assess the effect of the *Kwoniella mangroviensis* extract to protect the skin from extern injuries caused by pollution, first of all, it was performed a study of the viability of keratinocytes in the presence of increasing concentrations of Urban Dust, as disclosed in Fernandez et al., SIG-1273 protects skin against urban air pollution and when formulated in AgelQ™ Night Cream anti-aging benefits clinically demonstrated, J. Cosmet. Dermatol., 2019, doi: 10.1111/jocd.12825.

The viability of human epidermal keratinocytes (Sterlab Cat#205CELLK-N; Sterlab, Vallauris, France) was determined by measuring ATP. 25,000 cells in 100 µl. After 24 h, subconfluent cells were stimulated for 24 h with increasing concentrations of Urban Dust (0, 10, 50, 100, 200 and 500 µg/mL). All conditions were tested in triplicate. ATP measurement was performed according to the instructions provided by the commercial company (CellTiter-Glo^{®} from Promega; Madison, Wl, USA).

Urban Dust induces toxicity in human keratinocytes in a dose-dependent manner, exhibiting a statistically significant decrease in viability from a dose of 200 µg/ml. Although the trend is evident at lower doses, as shown in Figure 2.

It was also found that the extract itself did not induce toxicity in human keratinocytes. Thus, in Figure 3, it can be observed that, effectively, the extract did not induce any decrease in the viability of these cells, determined according to the above-mentioned method used to assess viability after exposition to Urban Dust.

Once the toxicities produced by the Urban Dust were verified, measurements of ROS (reactive oxygen species) and inflammatory mediators were performed.

ROS were measured in epidermal keratinocytes (Sterlab Cat#205CELLK-N; Sterlab, Vallauris, France) using the DCFH-DA reagent (Dichlorofluorescein diacetate, Invitrogen #C6827; Carlsbad, CA, USA). 25,000 cells were seeded in wells of 96-well plates. When 80% confluency was reached, cells were incubated with 10 µM DCFH-DA in 100 µl HANKS medium for 40 min at 37°C. Then, the cells were washed with 100 µl of HANKS medium and incubated with the products (Urban Dust 200 µg/mL without and with the active ingredient at different concentrations) for 2 h, after which the fluorescence measurement in the reader was carried out at wavelengths of 485/527 nm.

In Figure 4 it is shown that Urban Dust at 200 µg/ml induces an increase, with respect to the unstimulated control, of 164% in the formation of ROS. When the extract of the present invention was used together with Urban Dust 200 µg/ml at concentrations of 0.125 wt%, 0.25 wt% and 0.5 wt%, a statistically significant prevention was observed in the three tested doses.

To assess the impact of the extract on the induction of inflammation using Urban Dust a panel of inflammatory cytokines were measured.

This measurement was performed by qPCR using RPS28 gene-specific primers. RNA reverse transcription was performed using iScript cDNA Synthesis Kit (BioRad, Hercules, CA, USA) and relative quantification of gene expression was performed using the CFX Connect real-time RT-PCR thermal cycler (BioRad, Hercules, CA, USA) and SYBR Green (SsoAdvanced Universal SYBR Green, BioRad, Hercules, CA, USA), according to the manufacturer's protocol. The value of change of expression levels was calculated using the formula 2^{-ΔΔCT}, as disclosed in Livak et al., Analysis of relative gene expression data using real-time quantitative PCR and the 2-ΔΔCT Method., Methods, 2001, 25(4). 402-8, 2001, doi: 10.1006/meth.2001.1262.

In Figure 5, it can be observed that Urban Dust induced large increases in the 5 inflammatory genes CCL5, COX2, CSF2, GRO1 and TNFA.

However, when co-stimulating with Urban Dust and the extract, it can be observed that increasing levels of extract prevent markedly the expression of these 5 inflammatory cytokines.

### Example 4: Anti-aging effect of the Kwoniella mangroviensis extract

To assess the anti-aging affect, a massive sequencing experiment was carried out on human fibroblasts (HDF), both young HDF and two models of aged fibroblasts, the Hayflick model (Kim et al., op. cit.) and the H₂O₂ chemical induction model (De Magalhães *et al.*, *op. cit.*)*.*

The experiment was carried out either treating the cells with the extract (treated samples) or using the same cell models without treating the cells with the extract (control samples). RNA extraction from treated HDF cells was performed using TriPure Isolation Reagent^{®}, following the manufacturer's instructions. A minimum of 1 µg of RNA was obtained from each sample at a concentration > 75 ng/µl. Once the RNA samples were obtained, they were sequenced using the Illumina platform. The data was analysed using bioinformatic tools.

The expression level of each gene is compared between the HDFs in their basal state and in the two models of senescence, in the presence and absence of the active agent in question.

It was observed that the treatment with the extract induces the expression of genes related to the organization, structure and composition of the extracellular matrix (see Figure 6).

It was observed also that the presence of the extract reverses the effects caused by senescence in gene expression levels, wherein the genes are related to the processes of general senescence, and specific senescence in the skin (see Figure 7).

### Example 5: Cosmetic compositions

In this example are shown different cosmetic compositions comprising the cosmetic active ingredient of the invention as prepared according to Example 1.

### 5.1.) Facial serum

| **Ingredient** | **wt% in formula** |
|---|---|
| Deionized water | 90.9 |
| 1,2-hexanediol | 2.5 |
| Potassium sorbate | 0.2 |
| Glyceryl caprylate and glyceryl undecylenate | 0.9 |
| Citric acid sol 20% | 0.2 |

| **Cosmetic active ingredient (Example 1)** | **2** |
|---|---|
| Xanthan gum | 0.3 |
| Pentylene glycol | 2 |
| Polyacrylate Crosspolymer-6 | 0.8 |
| Sodium hydroxide sol (20%) | 0.2 |

### 5.2.) Comfort creamy ampoule

| **Ingredient** | **wt% in formula** |
|---|---|
| Water (aqua) | 91.40 |
| Microcrystalline cellulose and cellulose gum | 1.00 |
| 1,3-propanediol | 3.00 |

| **Cosmetic active ingredient (Example 1)** | **2.00** |
|---|---|
| Glyceryl caprylate and glyceryl undecylenate | 1.00 |
| Glyceryl stearate citrate | 0.20 |
| Polyglyceryl-6 stearate and polyglyceryl-6 behenate | 0.25 |
| Polyglyceryl-4 diisostearate/Polyhydroxystearate/Sebacate | 1.00 |
| Fragrance (Parfum) | 0.15 |

### 5.3.) Frosty hydraboost

| **Ingredient** | **wt% in formula** |
|---|---|
| Water (aqua) | 87.64 |
| Glycerin | 2.00 |
| 1,3-propanediol | 3.00 |
| Sodium Carbomer | 1.00 |

| **Cosmetic active ingredient (Example 1)** | **2.00** |
|---|---|
| Dicaprylyl ether | 3.00 |
| Glyceryl caprylate and glyceryl undecylenate | 1.00 |
| Fragrance (parfum) | 0.10 |
| Cl 74160, Water(aqua), glycerin, ceteareth-25, iodopropynyl butylcarbamate, phenoxyethanol | 0.01 |
| Water (aqua), sodium hydroxide | 0.25 |

## Claims

1. A cosmetic active ingredient comprising an extract of *Kwoniella mangroviensis.*

2. The cosmetic active ingredient according to claim 1, **characterized in that** the extract of *Kwoniella mangroviensis* comprises a supernatant and a lysate of the yeast cells obtained by culturing said cells in a culture medium.

3. The cosmetic active ingredient according to claim 1 or 2, **characterized in that** the extract further comprises an additive.

4. The cosmetic active ingredient according to claim 3, **characterized in that** the additive is selected from pentylene glycol, levulinic acid, glyceryl caprylate, sodium benzoate, potassium benzoate, sodium propionate, potassium sorbate, gluconolactone (δ-lactone of gluconic acid), lactic acid, tartaric acid, and mixtures thereof.

5. The cosmetic active ingredient according to claim 4, **characterized in that** the additive is a mixture of pentylene glycol, levulinic acid, and glyceryl caprylate.

6. The cosmetic active ingredient according to any one of claims 1 to 5, **characterized in that** it further comprises a cosmetically acceptable component selected from co-solvents, thickeners, additives, and mixtures thereof.

7. The cosmetic active ingredient according to claim 6, **characterized in that** it comprises an additive.

8. The cosmetic active ingredient according to claim 7, **characterized in that** the additive is selected from pentylene glycol, levulinic acid, glyceryl caprylate, sodium benzoate, potassium benzoate, sodium propionate, potassium sorbate, gluconolactone (δ-lactone of gluconic acid), lactic acid, tartaric acid, and mixtures thereof.

9. The cosmetic active ingredient according to claim 1, **characterized in that** it comprises the extract of *Kwoniella mangroviensis,* a co-solvent, preferably 1,3-propanediol, a thickener, preferably xanthan gum, and an additive, preferably a combination of pentylene glycol, levulinic acid, and glyceryl caprylate.

10. A cosmetic use of the cosmetic active ingredient according to any one of claims 1 to 9 for dermal application for improving the state of the skin.

11. The cosmetic use according to claim 10, **characterized in that** it is selected from for inducing a positive balance in the microbiome of the skin, protecting the skin from extern injuries caused by pollution, reversing the effects caused by ageing, and combinations thereof.

12. A cosmetic composition for topical application comprising the cosmetic active ingredient according to any one of claims 1 to 9 and at least one cosmetically acceptable component.

13. A cosmetic method for improving the state of the skin, preferably human skin, comprising applying topically to the skin of a subject in need thereof of an effective amount of a cosmetic composition comprising the cosmetic active ingredient according to any one of claims 1 to 9.

14. The cosmetic method according to claim 13, **characterized in that** the method for treating the skin is for improving the state of the skin selected from inducing a positive balance in the microbiome of the skin, protecting skin from extern injuries caused by pollution, reversing the effects caused by ageing, and combinations thereof.

15. The cosmetic method according to claim 13 or 14, **characterized in that** the cosmetic composition comprising the cosmetic active ingredient according to any one of claims 1 to 9 is applied at least once a day for at least 15, 28 or 56 days.
